# EUROPÄISCHE PATENTSCHRIFT

(11) **EP 1 526 838 B1**
(45) Veröffentlichungstag und Bekanntmachung des Hinweises auf die Patenterteilung: **22.08.2012**
(21) Anmeldenummer: 03766361.4
(22) Anmeldetag: 30.07.2003
(51) Int. Cl.: A61K 9/10

(54) **INTRAVEN S APPLIZIERBARE, PHARMAZEUTISCHE DARREICHUNGSFORM**
INTRAVENOUS PHARMACEUTICAL FORM OF ADMINISTRATION
POSOLOGIE PHARMACEUTIQUE A ADMINISTRATION INTRAVEINEUSE

(30) Priorität: 30.07.2002 DE 10234784
(43) Veröffentlichungstag der Anmeldung: 04.05.2005
(73) Patentinhaber: Grünenthal GmbH, 52078 Aachen (DE)
(72) Erfinder: KUGELMANN, Heinrich, 52068 Aachen (DE); BARTHOLOMÄUS, Johannes, 52080 Aachen (DE)
(74) Vertreter: Kutzenberger, Helga
(86) Internationale Anmeldenummer: PCT/EP2003/008421
(87) Internationale Veröffentlichungsnummer: WO 2004/012708

(56) Entgegenhaltungen:
- WO-A-91/14455
- DE-A- 4 440 337
- M. EANDI ET AL.: "Absolute bioavailability of paracetamol after oral or rectal administration in healthy volunteers" ARZNEIMITTEL-FORSCHUNG. DRUG RESEARCH., Bd. 34, Nr. 8, August 1984 (1984-08), Seiten 903-907, XP002259247 Aulendorf (DE)

## Beschreibung

Die vorliegende Erfindung betrifft eine intravenös applizierbare, pharmazeutische Darreichungsform sowie ein Kit enthaltend die Komponenten zur Herstellung einer solchen Darreichungsform.

In einer Vielzahl von Fällen ist die zufriedenstellende Behandlung eines Patienten mit einem pharmazeutischen Wirkstoff nur über die parenterale Verabreichung des Wirkstoffs zu erreichen. Dies ist beispielsweise der Fall, wenn der Patient aufgrund seiner körperlichen Beeinträchtigung nicht oder nur schlecht in der Lage ist, den jeweiligen Wirkstoff oral einzunehmen, der Wirkstoff über den Darm nur unzureichend resorbiert wird oder wenn der Wirkstoff gegenüber der Magensäure oder den Enzymen des Verdauungstraktes nicht ausreichend beständig ist. Ferner ist die parenterale Verabreichung eines Wirkstoffs auch dann vorteilhaft, wenn eine schnelle, direkte Wirkung des Wirkstoffs erreicht werden soll.

Eine besondere Form der parenteralen Verabreichung stellt die intravenöse Applikation dar, bei der der Wirkstoff mit Hilfe einer Injektion oder Infusion in eine Vene des Patienten appliziert wird. Während bei der intravenösen Injektion die gesamte Wirkstoffmenge unmittelbar nach der Applikation im Organismus zur Verfügung steht, bietet die intravenöse Infusion die Möglichkeit die gesamte Wirkstoffmenge über einen längeren Zeitraum zu verabreichen.

Die intravenöse Applikation von pharmazeutischen Wirkstoffen erfolgt nahezu ausschließlich über eine wäßrige Lösung des jeweiligen Wirkstoffs, da die Mischbarkeit der applizierten Lösung mit dem Blut des Patienten eine wesentliche Voraussetzung für eine gefahrlose Verabreichung darstellt. Ist diese Mischbarkeit nicht gegeben, drohen dem Patienten lebensgefährliche Embolien oder schwere Nekrosen bis hin zu einer Amputation des entsprechenden Gliedmaßes. Zwar sind auch intravenös applizierbare Öl in Wasser Emulsionen bekannt, die einen lipophilen Wirkstoff in der dispersen Phase aufweisen. Das Aufnahmevermögen solcher Emulsionen für den lipophilen Wirkstoff ist aber zum einen durch die Löslichkeit des Wirkstoffs in der Ölphase und zum anderen durch die physikalische Stabilität der Emulsion begrenzt, so daß diese Form der Darreichung keine weite Verbreiteng erfahren hat.

Die intravenöse Applikation von Wirkstoffen ist daher üblicherweise auf solche Wirkstoffe beschränkt, die sich durch eine ausreichend hohe Wasserlöslichkeit auszeichnen. Diese gewährleistet, daß eine, für eine zufriedenstellende Behandlung des Patienten erforderliche Menge des Wirkstoffs in dem zu applizierenden wäßrigen Medium vollständig gelöst werden kann. Zahlreiche Wirkstoffe mit schlechter bis sehr schlechter Wasserlöslichkeit können dagegen üblicherweise nur auf anderem Wege, beispielsweise oral oder rektal, an den Patienten verabreicht werden, da das Volumen des wäßrigen Mediums, das für die vollständige Lösung des Wirkstoffes erforderlich wäre, eine intravenöse Applikation nicht mehr zuläßt. Die orale oder rektale Applikation hat aber u.a. den Nachteil, daß zumeist höhere Wirkstoffmengen zu verabreichen sind, als es bei einer intravenösen Applikation des jeweiligen Wirkstoffes für eine zufriedenstellende Behandlung des Patienten erforderlich wäre. Sofern dennoch Wirkstoffe mit schlechter Wasserlöslichkeit, die zu einem unlöslichen Anteil in einem für eine intravenöse Applikation üblichen Volumen eingesetzt werden, wurde für den ungelösten Wirkstoffanteil immer nur eine durchschnittliche Partikelgröße im Nanometerbereich als zulässig und tolerierbar, weil unverzüglich löslich bei der Applikation, angesehen. Eine solche Aufbereitung des Wirkstoffes zu Partikeln im Nanometerbereich ist aber aufwendig und kostspielig.

NO 91/14455 beschreibt wässrige Suspensionen zur epiduralen und intrathehalen Injection von Wirkstoffen zur Schmerzbehampfung.

Die der vorliegenden Erfindung zugrundeliegende Aufgabe bestand daher darin, eine intravenös applizierbare, pharmazeutische Darreichungsform auch für einen Wirkstoff mit ungenügender Wasserlöslichkeit zur Verfügung zu stellen, die eine für eine zufriedenstellende Behandlung des Patienten ausreichende Menge an Wirkstoff enthält und deren in Wasser unlöslicher Wirkstoffanteil nicht nur Partikel im Nanometerbereich, aufweisen darf, um so u. a. die aufwendige Vorbereitung der Wirkstoffe zu vermeiden.

Erfindungsgemäß wird diese Aufgabe durch die Bereitstellung einer intravenös applizierbaren, pharmazeutischen Darreichungsform gelöst, die einen oder mehrere pharmazeutische Wirkstoffe jeweils zumindest teilweise ungelöst in einem auf Wasser basierenden Suspensionsmedium aufweist und dieser ungelöste Wirkstoffanteil eine durchschnittliche Partikelgröße von ≥ 5 µm hat, wobei davon der Anteil an Partikeln mit einer Partikelgröße im Bereich von größer 2 µm - 100 µm mindestens 80% der Gesamtmasse der Partikel beträgt und dieser ungelöste Wirkstoffanteil maximal so groß ist, daß er sich bei Verdünnung mit bis zu 500 ml Phosphatpufferlösung bei Körpertemperatur, vorzugsweise ≥ 35°C, innerhalb einer vorgegebenen Applikationszeit löst.

Vorzugsweise liegt die durchschnittliche Partikelgröße der ungelösten Wirkstoffpartikel im Bereich von > 5 µm bis 35 µm, wobei davon vorzugsweise der Anteil an Partikeln mit einer Partikelgröße im Bereich von 3 - 80 µm, vorzugsweise 3 - 50 µm, mindestens 80% der Gesamtmasse der Partikel beträgt.

Die intravenös applizierbare, pharmazeutische Darreichungsform gemäß der vorliegenden Erfindung eignet sich sowohl zur Verabreichung pharmazeutischer Wirkstoffe an Menschen wie auch an Tiere. Bevorzugt eignet sie sich zur Verabreichung pharmazeutischer Wirkstoffe an den Menschen.

Die Körpertemperatur variiert, abgesehen von den geringen natürlichen Schwankungen zwischen verschiedenen Vertretern einer Spezies, z.B. auch in Abhängigkeit von der jeweils zu behandelnden Spezies, beispielsweise Mensch oder Tier, oder in Abhängigkeit von dem jeweiligen Zustand, wie z.B. Unterkühlung oder Fieber.

Dem Fachmann sind die entsprechenden Temperaturbereiche, die für die jeweilige Spezies, insbesondere für den Menschen in Betracht kommen, bekannt. Vorzugsweise ist die entsprechende Temperatur die jeweilige Körpertemperatur des zu behandelnden Menschen besonders bevorzugt ≥ 35°C.

Die Applikationszeit hängt von den Löslichkeitseigenschaften des ungelösten Wirkstoffanteils in dem vorgegebenen Volumen der Phosphatpufferlösung und dem Ort der Applikation, z.B. Arm, im wesentlichen ab. Geeignete Methoden zur Bestimmung der notwendigen Applikationszeit in Abhängigkeit vom Applikationsort sind dem Fachmann an sich bekannt und beispielsweise in Physiologie des Menschen, R. F. Schmidt, G. Thews, Springer Verlag Berlin, Heidelberg, New York, 20. Auflage 1980, beschrieben. Die entsprechende Literturbeschreibung wird hiermit als Referenz eingeführt und gilt als Teil der Offenbarung.

Die für die jeweilige erfindungsgemäße Darreichungsform notwendige Applikationszeit kann vom Fachmann durch einfache Vorversuche mit Hilfe einer Messung in dem nachstehend beschriebenen Modell ermittelt werden, wobei zur Vermeidung jedes gesundheitlichen Risikos die so ermittelte Applikationszeit um mindestens 10 %, bis maximal 100 % zu verlängern ist.

Zur Bestimmung der Applikationszeit wird zunächst mit Hilfe einer Standardphosphatpufferlösung mit einem pH-Wert von 7,413 (bei 25 °C) die notwendige Löslichkeit des ungelösten Wirkstoffanteils bestimmt. Der pH-Wert dieser Standardpufferlösung bei einer Temperatur von 37 °C kann nach üblichen, dem Fachmann bekannten Methoden bestimmt werden. Die entsprechende Standardpufferlösung ist beispielsweise von der Firma Riedel-de Haen, Hannover, Deutschland, am Markt erhältlich.

Die erfindungsgemäße, intravenös applizierbare Darreichungsform kann sowohl in Form einer Injektion als auch in Form einer Infusion vorliegen. Vorzugsweise liegt die erfindungsgemäße, intravenös applizierbare Darreichungsform in Form einer Injektion vor.

Das Suspensionsvolumen der erfindungsgemäßen, intravenös applizierbaren Darreichungsform sollte in einer für diese Form der Darreichung üblichen, dem Fachmann bekannten Größenordnung liegen, sofern die vorstehend angegebenen erfindungsgemäßen Bedingungen erfüllt sind.

Sofern die erfindungsgemäße Darreichungsform zur intravenösen Applikation über eine Injektion vorgesehen ist, beträgt das Suspensionsvolumen bevorzugt 0,1 bis 15 ml, besonders bevorzugt 0,5 bis 10 ml.

Ist die erfindungsgemäße Darreichungsform zur intravenösen Applikation über eine Infusion vorgesehen, beträgt das Volumen der intravenös applizierbaren Suspension vorzugsweise >15 bis 500 ml, besonders bevorzugt 50 bis 250 ml.

In der erfindungsgemäßen Darreichungsform weist der Anteil an ungelösten Wirkstoffpartikeln in der intravenös zu applizierenden Suspension eine durchschnittliche Partikelgröße > 5 µm, vorzugsweise im Bereich von > 5 bis 35 µm auf, wobei der Anteil an Partikeln mit einer Partikelgröße im Bereich von > 2 µm - 100 µm, vorzugsweise im Bereich von 3 - 80 µm, besonders bevorzugt im Bereich von 3 -50 µm, mindestens 80% der Gesamtmasse dieser Wirkstoffpartikel beträgt. Diese Partikelgrößen der ungelösten Wirkstoffpartikel werden über eine Laser-Streulichtmessung in einem Coulter^{®} LS 230 Laserpartikelanalysator mit HFM- und MVM-Modul (Beckman-Coulter Electronics GmbH, Krefeld, Germany) durch Auswertung nach dem Partikelvolumen bestimmt.

Als Suspensionsmedium für die Messung der jeweiligen Partikelgrößen wird eine isotonische Natriumchloridlösung mit einem Zusatz von 0,1 Gew.% Polysorbat 80 verwendet, in der der Wirkstoff in Pulverform durch Schütteln suspendiert wird. Die Messung wird unmittelbar nach der Suspendierung durchgeführt.

Die erfindungsgemäße, intravenös applizierbare Darreichungsform kann einen oder mehrere pharmazeutische Wirkstoffe aufweisen, sofern diese den oben angegebenen Bedingungen genügen. Vorzugsweise enthält die erfindungsgemäße, intravenös applizierbare Darreichungsform jeweils nur einen pharmazeutischen Wirkstoff, dessen zu verabreichende Dosis in dem Applikationsvolumen zumindest teilweise ungelöst, vorzugsweise suspendiert vorliegt.

Als pharmazeutische Wirkstoffe kann die erfindungsgemäße, intravenös applizierbare Darreichungsform jeden pharmazeutischen Wirkstoff enthalten, der den oben angegebenen Bedingungen genügt, d.h. in dem zu applizierenden Volumen mit der zu verabreichenden Wirkstoffdosis zwar nicht vollständig löslich ist, aber sich bei Verdünnung mit bis zu 500 ml der genannten Phosphatpufferlösung bei Körpertemperatur innerhalb der Applikationszeit löst, vorzugsweise molekulardispers löst, ohne daß die ungelösten Partikel eine durchschnittliche Partikelgröße im Nanometerbereich aufweisen müssen. Dabei eignet sich die erfindungsgemäße Darreichungsform insbesondere für Wirkstoffe, die mittel bis schwer löslich in Wasser sind.

Vorzugsweise enthält die erfindungsgemäße Darreichungsform einen oder mehrere Wirkstoffe ausgewählt aus der Gruppe bestehend aus Analgetika, Antiadiposita, Analeptika, Antihypoxämika, Antirheumatika, Opioid-Antagonisten, Anthelminika, Antiallergika, Antiarrhythmika, Antibiotika, Antidementiva (Nootropica), Antidiabetika, Antiemetika, Antivertiginosa, Antiepileptika, Antihypertonika, Antihypotonika, Antimykotika, Antiphlogistika, Antitussiva, Expectorantia, Arteriosklerosemittel, β-Rezeptorenblocker, Kalziumkanalblocker, Broncholytika, Antiasthmatika, Cholinergika, Diuretika, durchblutungsfördernden Mittel, Entwöhnungsmittel, Geriatrika, Hypnotika, Sedativa, Immunmodulatoren, Mundtherapeutika, Rachentherapeutika, Koronarmittel, Lipidsenker, Lokalanästhetika, Neuraltherapeutika, Magenmittel, Darmmittel, Migränemittel, Muskelrelaxantia, Narkosemittel, Neuropathiepräparate, Ophthalmika, Otologika, Parkinsonmittel, Psychopharmaka, Rhinologika, Sinusitismittel, Spasmolytika, Thrombocytenaggregationsmemmer, Tuberculosemittel, Urologica und Zystostatika.

Besonders bevorzugt ist der pharmazeutische Wirkstoff ausgewählt aus der Gruppe bestehend aus Analgetika, Analeptika, Antihypoxämika, Antiallergika, Antiarrhythmika, Antiemetika, Antivertiginosa, Antihypertonika, Antihypotonika, Antitussiva, Expectorantia, β-Rezeptorenblocker, Kalziumkanalblocker, Ophthalmika, Otologika, Spasmolytika und Urologica, ganz besonders bevorzugt aus der Gruppe der Analgetika, wie beispielsweise Paracetamol.

Die erfindungsgemäße, intravenös applizierbare Darreichungsform kann den (die) jeweiligen Wirkstoff(e) auch in Form einer entsprechenden physiologisch verträglichen Verbindung, vorzugsweise in Form eines entsprechenden physiologisch verträglichen Salzes oder Solvatverbindung enthalten, sofern diese Verbindung den oben genannten Bedingungen genügt. Der Wirkstoff wird zur Zubereitung der erfindungsgemäßen Darreichungsform in Pulverform gebracht.

Als flüssiges Suspensionsmedium enthält die erfindungsgemäße, intravenös applizierbare Darreichungsform Wasser oder ein auf Wasser basierendes Medium.

Neben Wasser kann das Suspensionsmedium dabei auch übliche, dem Fachmann bekannte physiologisch verträgliche Hilfsstoffe enthalten. Vorzugsweise sind diese physiologisch verträglichen Hilfsstoffe ausgewählt aus der Gruppe bestehend aus pH-Regulatoren, Regulatoren zur Einstellung der Osmolalität, grenzflächenaktiven Verbindungen, Viskositätsregulatoren, Peptisatoren, Puffern und Konservierungsmitteln.

Neben einem oder mehreren Vertretern einer Hilfsstoffklasse kann das Suspensionsmedium auch einen oder mehrere Vertreter einer bis aller übrigen genannten Hilfsstoffklassen enthalten.

Sofern die erfindungsgemäße, intravenös applizierbare Darreichungsform physiologisch verträgliche grenzflächenaktive Verbindungen enthält, sind dies bevorzugt Polyalkylenglykole, wie beispielsweise Polyethylenglykole, Polypropylenglykole oder Ethylenoxid/Propylenoxid-Blockcopolymere, Phospholipide, Ether oder Ester von gesättigten oder ungesättigten Fettalkoholen oder Fettsäuren mit Polyalkylenglykolen, wie beispielsweise Polyethylenglykolen oder Polypropylenglykolen, Polysorbate, wie Mono-, Di-, oder Triester von gesättigten oder ungesättigten Fettsäuren, bevorzugt Ölsäure, Laurinsäure, Palmitinsäure oder Stearinsäure, und Sorbitol und/oder seinem Anhydrid, die bis zu 20 Mol Ethylenoxid-Einheiten pro Mol Sorbitol bzw. Anhydrid aufweisen können, vorzugsweise Polyethoxysorbitanmonolaurat mit 20 Ethylenoxid-Einheiten, Polyethoxysorbitanmonolaurat mit 4 Ethylenoxid-Einheiten, Polyethoxysorbitanmonopalmitat mit 20 Ethylenoxid-Einheiten, Polyethoxysorbitanmonostearat mit 20 Ethylenoxid-Einheiten, Polyethoxysorbitanmonostearat mit 4 Ethylenoxid-Einheiten, Polyethoxysorbitantristearat mit 20 Ethylenoxid-Einheiten, Polyethoxysorbitanmonooleat mit 20 Ethylenoxid-Einheiten, Polyethoxysorbitanmonooleat mit 5 Ethylenoxid-Einheiten oder Polyethoxysorbitantrioleat mit 20 Ethylenoxideinheiten. Es kann auch ein Gemisch aus wenigstens zwei Vertretern aus verschiedenen, vorstehend genannten Klassen von grenzflächenaktiven Verbindungen oder aus wenigstens zwei Vertretern aus einer Klasse von grenzflächenaktiven Verbindungen zum Einsatz kommen.

Eine Vielzahl der entsprechenden Polysorbate wird unter dem Handelsnamen Tween^{®} von der Firma ICI Surfactants (Essen, Deutschland) am Markt geführt.

Um das Risiko von Zell- und Gewebeschädigungen bei der intravenösen Applikation der erfindungsgemäßen Darreichungsform zu minimieren bzw. völlig auszuschließen, wird die Osmolalität, d.h. die Tonizität der erfindungsgemäßen Darreichungsform bevorzugt so eingestellt, daß sie isotonisch oder zumindest annähernd isotonisch zu der physiologischen Osmolalität ist. Bevorzugt wird daher die Osmolalität der erfindungsgemäßen, intravenös applizierbaren Darreichungsform so eingestellt, daß sie im Bereich von 250 bis 400 mOsm/kg, besonders bevorzugt im Bereich von 260 bis 320 mOsm/kg und ganz besonders bevorzugt im Bereich von 280 bis 300 mOsm/kg liegt.

Bevorzugte Regulatoren zur Einstellung der Osmolalität sind wasserlösliche, physiologisch verträgliche Verbindungen wie anorganische Salze, z. B. Alkalisalze, bevorzugt Natriumchlorid, Zucker, z.B. Saccharose oder Dextrose, Zuckeralkohole, z.B. Mannitol, oder Polyalkylenglykole, z.B. Polyethylenglykole, vorzugsweise solche mit einem Molekulargewicht von 1000 bis 8000 g/mol. Es kann auch ein Gemisch aus wenigstens zwei Vertretern aus verschiedenen, vorstehend genannten Regulatorenklassen oder wenigstens zwei Vertretern aus einer Regulatorenklasse zur Einstellung der Osmolalität verwendet werden.

Gegebenenfalls kann ein Hilfsstoff auch zur Einstellung verschiedener Eigenschaften der erfindungsgemäßen, intravenös applizierbaren Darreichungsform eingesetzt werden. Beispielsweise kann eine grenzflächenaktive Verbindung auch zur Einstellung der Osmolalität dienen.

Der pH-Wert der erfindungsgemäßen, intravenös applizierbaren Darreichungsform sollte vorzugsweise im Bereich von pH 5 bis pH 8 liegen, um das Risiko von Zell- und Gewebeschädigungen zu minimieren bzw. völlig auszuschließen. Die Einstellung des pH-Wertes der erfindungsgemäßen Darreichungsform kann nach üblichen, dem Fachmann bekannten Methoden erfolgen.

Die erfindungsgemäße, intravenös applizierbare Darreichungsform kann auch physiologisch verträgliche Konservierungsmittel enthalten. Als solche eignen sich beispielsweise 1,1,1-Trichlor-2-methyl-2-propanol, Phenylethylalkohol, Sorbinsäure, Benzylalkohol, Alkylbenzyldimethylammoniumchlorid mit einer Kettenlänge von C₈ bis C₁₈ im Alkylteil, m-Kresol oder 4-Hydroxyalkylbenzoat, vorzugsweise 4-Hydroxymethylbenzoat oder 4-Hydroxypropylbenzoat. Es können auch Mischungen aus zwei oder mehreren der vorstehend genannten physiologisch verträglichen Konservierungsmittel zum Einsatz kommen.

Ein weiterer Gegenstand der vorliegenden Erfindung ist ein Kit zur Herstellung der erfindungsgemäßen, intravenös applizierbaren Darreichungsform, welches die zur Herstellung dieser Darreichungsform erforderlichen Komponenten zumindest teilweise separiert voneinander enthält.

In einer bevorzugten Ausführungsform besteht dieses Kit aus zwei Gefäßen, wobei ein Gefäß das Suspensionsmedium und das andere Gefäß den/die pharmazeutischen Wirkstoff(e), vorzugsweise in Pulverform, enthält.

Gemäß einer bevorzugten Ausführungsform kann wenigstens eines dieser Gefäße eine Ampulle oder Flasche (vial) sein, wobei vorzugsweise ein Gefäß an das andere zum Mischen andockbar ist.

In einer weiteren bevorzugten Ausführungsform liegt das Kit in Form einer Zweikammerspritze vor, wobei eine Kammer das Suspensionsmedium und die andere Kammer die zu suspendierenden Wirkstoffpartikel enthält.

Sofern die obenstehend genannten Bedingungen erfüllt sind, kann die jeweils an den Patienten zu verabreichende Menge des jeweiligen Wirkstoffes bzw. der jeweiligen Verbindung dieses Wirkstoffes in der erfindungsgemäßen, intravenös applizierbaren Darreichungsform variieren, beispielsweise in Abhängigkeit vom Gewicht des Patienten, der Art der zu therapierenden Beschwerden oder der Krankheit. Dem Fachmann ist aufgrund der Eigenschaften der jeweiligen Wirkstoffe bekannt, in welchen Dosierungen diese einzusetzen sind um die gewünschte therapeutische Wirkung zu erzielen.

Die Herstellung der erfindungsgemäßen, intravenös applizierbaren Darreichungsform bzw. ihrer Komponenten kann nach üblichen, dem Fachmann bekannten Methoden erfolgen. Sofern die erfindungsgemäße, intravenös applizierbare Darreichungsform bereits darreichungsfertig formuliert wird, kann die Herstellung bevorzugt mit Hilfe der nachstehend beschriebenen Methode erfolgen.

Sofern feste physiologisch verträglich Hilfsstoffe zum Einsatz kommen, werden diese bei Raumtemperatur, circa 15 bis 25 °C, oder gegebenenfalls unter Erwärmen in Wasser für Injektionszwecke gelöst. Handelt es sich um flüssige Hilfsstoffe, werden diese mit dem Wasser vermischt. Die so erhaltene Lösung bzw. Mischung wird anschließend unter Verwendung eines Filters, welcher Mikroorganismen zurückhält, steril filtriert. Üblicherweise beträgt die Porenweite des Filters 0,2 µm. Die Filtration kann gegebenenfalls auch vor der Zugabe der physiologisch verträglichen Hilfsstoffe erfolgen, jedoch sollte dann die weitere Herstellung der erfindungsgemäßen Darreichungsform unter aseptischen Bedingungen erfolgen.

Anschließend wird das sterile pharmazeutische Wirkstoffpulver unter aseptischen Bedingungen durch Rühren in das so erhaltene Suspensionsmedium eingebracht und die so erhaltene Suspension anschließend in geeignete Behälter, vorzugsweise in Injektionsflaschen (vials) oder Infusionsflaschen abgefüllt.

Sofern die Herstellung der erfindungsgemäßen, intravenös applizierbaren Darreichungsform nicht bereits unter aseptischen Bedingungen durchgeführt wurde, kann gegebenenfalls eine Endsterilisation nach üblichen, dem Fachmann bekannten Methoden, beispielsweise durch Autoklavieren, vorgenommen werden. Vorzugsweise wird aber bereits die erfindungsgemäße, intravenös applizierbare Darreichungsform unter aseptischen Bedingungen hergestellt.

Mit Hilfe der erfindungsgemäßen intravenös applizierbaren, pharmazeutischen Darreichungsform ist es möglich, vorzugsweise pharmazeutische Wirkstoffe mit einer schlechten bis sehr schlechten Wasserlöslichkeit intravenös in einer für die notwendige Behandlung des Patienten ausreichenden Dosis zu verabreichen, ohne daß der Wirkstoff einer aufwendigen Behandlung zur Erzielung einer Partikelgröße im Nanometerbereich unterworfen werden muß.

Ferner können pharmazeutische Wirkstoffe mit einer guten Wasserlöslichkeit in einem geringeren Applikationsvolumen intravenös verabreicht werden, was insbesondere bei Infusionen u.a. den Vorteil hat, daß die bei der Verabreichung von großen Volumina auftretenden Gefahren, z.B. Lungenödemen, ausgeschlossen oder zumindest vermindert werden können.

Die Osmolalität der erfindungsgemäßen, intravenös applizierbaren Darreichungsform wird durch Gefrierpunktserniedrigung nach Pharm. Eur. 97 gemäß Kapitel 2.2.35 bestimmt. Die entsprechende Literaturbeschreibung wird hiermit als Referenz eingeführt und gilt als Teil der Offenbarung. Die Messung erfolgte mittels eines Meßgerätes vom Typ M (Dr. H. Knauer KG, Berlin, Deutschland).
Die Kalibrierung wurde mit destilliertem Wasser für 0 mOsmoi/kg sowie mit einer Eichlösung (Dr. H. Knauer KG, Berlin, Deutschland) oder alternativ 12,687 g Natriumchlorid, gelöst in 1 kg destilliertem Wasser für 400 mOsmol/kg durchgeführt.

Zur Herstellung von Phosphatpufferlösung mit einem pH-Wert von 7,413 (bei 25 °C) werden Kaliumdihydrogenphosphat und Dinatriumhydrogenphosphat vor der Einwaage 2 Stunden bei 110-130 °C getrocknet und im Exsikkator abgekühlt. Anschließend werden 1,179 g Kaliumdihydrogenphosphat und 4,30 g Dinatriumhydrogenphosphat in circa 800 ml Wasser gelöst und bei 25°C zu 1000 ml aufgefüllt.

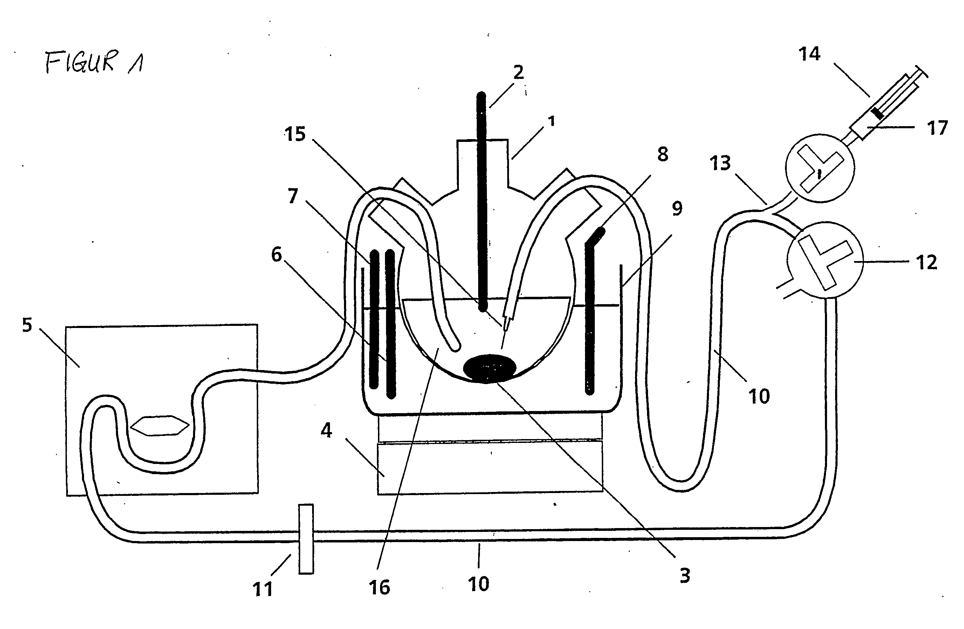

Bestimmung des Auflöseverhaltens des ungelösten Wirkstoffanteils im Kreislaufmodell:
Das Kreislaufmodell ist in Figur 1 wiedergegeben.

Dieses Kreislaufmodell simuliert in-vitro das menschliche Blutkreislaufsystem mit Gefäßsystem, Herz und Lunge. Als Prüfmedium wurde Phosphatpufferlösung mit einem pH-Wert von 7,413 (bei 25 °C) der oben angegebenen Zusammensetzung verwendet.

Der 500 ml Dreihalskolben (1) enthält 200 ml der vorstehend beschriebenen Phosphatpufferlösung (16) als Prüfmedium. Dieser Dreihalskolben simuliert das Herz. Der Dreihalskolben befindet sich in einer Temperierwanne (9), deren Temperatur auf 37 °C eingestellt wird. Der Zufluß (7) und Abfluß (8) der Temperierflüssigkeit zum Thermostat halten die Temperatur während der gesamten Untersuchung konstant auf 37 °C. Die Temperatur der Temperierflüssigkeit wird mit Hilfe des Temperaturfühlers (6), die Temperatur des Prüfmediums mit dem Temperaturfühler (2) kontrolliert und geregelt. Das Prüfmedium im Dreihalskolben wird mit einem Magnetrührstab (3) und Magnetrührer (4) gerührt. Im Dreihalskolben taucht ein Infusionsschlauch (10) in das Prüfmedium ein und wird durch eine seitliche Öffnung des Dreihalskolbens zu einer Peristaltikpumpe (5) geführt. Mit der Peristaltikpumpe (5) wird der Fluß des Prüfmediums innerhalb des Infusionsschlauchsystems (10) und zurück zum Dreihalskolben erreicht. Hinter der Peristaltikpumpe (5) wird ein Partikelfilter (11) mit einer Porenweite von 0,2 µm in das Infusionsschlauchsystem (10) eingebaut, der die Lunge simuliert. Hinter dem Partikelfilter (11) führt der Infusionsschlauch (10) zu einem Dreiwegehahn (12) und von dort zu einem Y-Stück (13). An der einen Seite des Y-Stücks führt der Infusionsschlauch weiter und durch die zweite seitliche Öffnung des Dreihalskolbens zurück und schließt somit den Kreislauf. Das Ende des Infusionschlauches endet unter dem Flüssigkeitsspiegel mit einer Kanüle (15). Auf der anderen Seite des Y-Stücks ist ein Dreiwegehahn und eine Spritze (14) angeschlossen. In der Spritze befindet sich die zu prüfende intravenöse Darreichungsform (17).

Bei geschlossenen Dreiwegehahn zur Spritze wird das Prüfmedium vom Dreihalskolben mittels der Peristaltikpumpe durch das Infusionschlauchsystem und den Filter zum Dreihalskolben zurückgepumpt. Für den Zeitraum des Zuspritzens der intravenösen Suspension wird der Dreiwegehahn zur Spritze geöffnet und die Suspension mit der Spritze zugespritzt. Nach dem Zuspritzen wird der Dreiwegehahn zur Spritze wieder geschlossen. Die Applikationsgeschwindigkeit und die daraus resultierende Applikationszeit für ein bestimmtes Volumen wird dadurch festgelegt, daß spätestens bei Eintritt in das Prüfmedium jegliche Trübung durch unlösliche Anteile nicht mehr gegeben ist. Darüber hinaus wird die komplette Auflösung durch Kontrolle des Partikelfilters (11) verifiziert.

Im folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1:

### Intravenös applizierbare Suspension von Paracetamol

Paracetamolpulver mit einer Partikelgröße von ca. 98 Gew.-% kleiner 50 µm und ca. 80 Gew.-% kleiner 10 µm wurde zu 1,0 g in vials aus Röhrenglas (15R) gefüllt, mit einem Injektionsstopfen aus Brombutylgummi verschlossen und mit einer Bördelkappe verbördelt.

Zur Suspendierung von 1 g Paracetamol wurden 5 ml isotonische Natriumchloridlösung mit einem Zusatz von 0,1 Vol % Polysorbat 80 (Gemisch aus Partialestern des Sorbitols mit Ölsäure und Sorbitol und/ oder seinem Anhydrid mit jeweils bis zu 20 Mol Ethylenoxid pro Mol Sorbitol und/ oder Sorbitolanhydrid) eingesetzt. Von dieser Suspension wurde unmittelbar nach ihrer Herstellung nach der vorstehend angegebenen Methode die Partikelgröße bestimmt. Die durchschnittliche Partikelgröße der ungelösten Partikel betrug > 5 µm, wobei der Anteil der Partikel mit einer Partikelgröße im Bereich von 6 - 40 µm 80% betrug.

### Beispiel 2:

### Intravenös applizierbare Suspension von Paracetamol

Paracetamol mit einem Partikeldurchmesser von ca. 98 Gew.-% kleiner 50 µm und ca. 80 Gew.-% kleiner 10 µm wurde zu 1,0 g in vials aus Röhrenglas (15R) gefüllt, mit einem Injektionsstopfen aus Brombutylgummi verschlossen und mit einer Bördelkappe verbördelt. Zur Suspendierung von 1 g Paracetamolpulver wurden 10 ml isotonische Natriumchloridlösung mit einem Zusatz von 0,1 Gew.-% Polysorbat 80, Gemisch aus Partialestern des Sorbitols und seiner Anhydride mit Ölsäure, Copolymerisat mit etwa 20 Mol Ethylenoxid für jeden Mol Sorbitol und Sorbitolanhydrid, eingesetzt.

Die Prüfung der Auflösung des ungelösten Anteils erfolgte mit dem vorstehend beschriebenen Kreislaufmodell. Beim Zuspritzen jeweils der erfindungsgemäßen Suspensionen gemäß Beispiel 1 und 2 löste sich das Paracetamol im Infusionsschlauch zum Dreihalskolben und im Dreihalskolben auf. Die den Dreihalskolben verlassende Prüfflüssigkeit enthielt keine ungelösten Bestandteile. Ein Umpumpen der Modellflüssigkeit war jederzeit möglich. Es trat kein Verstopfen des 0,2 µm-Partikelfilters (11) und damit ein Blockieren des Kreislaufes auf.

## Patentansprüche

1. Intravenös applizierbare, pharmazeutische Darreichungsform, **dadurch gekennzeichnet, daß** sie einen oder mehrere pharmazeutische Wirkstoffe jeweils zumindest teilweise ungelöst in einem auf Wasser basierenden Suspensionsmedium aufweist und dieser ungelöste Wirkstoffanteil eine mittleren durchschnittliche Partikelgröße von > 5 µm hat, wobei davon der Anteil an Partikeln mit einer Partikelgröße im Bereich von größer 2 µm - 100 µm mindestens 80% der Gesamtmasse dieser Partikel beträgt, und dieser ungelöste Wirkstoffanteil maximal so groß ist, daß er sich bei Verdünnung mit bis zu 500 ml Phosphatpufferlösung bei Körpertemperatur innerhalb einer vorgegebenen Applikationszeit löst.

2. Intravenös applizierbare, pharmazeutische Darreichungsform gemäß Anspruch 1, **dadurch gekennzeichnet, daß** sie zur intravenösen Injektion geeignet ist.

3. Intravenös applizierbare, pharmazeutische Darreichungsform gemäß Anspruch 1 oder 2, **dadurch gekennzeichnet, daß** die durchschnittliche Partikelgröße der ungelösten Wirkstoffpartikel im Bereich von > 5 bis 35 µm liegt, wobei der Anteil an Partikeln mit einer Partikelgröße im Bereich von 3 - 80 µm, vorzugsweise 3 - 50 µm mindestens 80% der Gesamtmasse dieser Partikel beträgt.

4. Intravenös applizierbare, pharmazeutische Darreichungsform gemäß einem der Ansprüche 1 bis 3, **dadurch gekennzeichnet, daß** die Wirkstoffpartikel, vorzugsweise erst unmittelbar vor der Applikation an den Patienten im Suspensionsmedium, suspendiert werden.

5. Intravenös applizierbare, pharmazeutische Darreichungsform gemäß einem der Ansprüche 1 bis 4, **dadurch gekennzeichnet, daß** der pharmazeutische Wirkstoff wenigstens ein Wirkstoff ausgewählt ist aus der Gruppe bestehend aus Analgetika, Antiadiposita, Analeptika, Antihypoxämika, Antirheumatika, Opioid-Antagonisten, Anthelminika, Antiallergika, Antiarrhythmika, Antibiotika, Antidementiva (Nootropica), Antidiabetika, Antiemetika, Antivertiginosa, Antiepileptika, Antihypertonika, Antihypotonika, Antimykotika, Antiphlogistika,
Antitussiva, Expectorantia, Arteriosklerosemittel, β-Rezeptorenblocker, Kalziumkanalblocker, Broncholytika, Antiasthmatika, Cholinergika, Diuretika, durchblutungsfördernde Mittel, Entwöhnungsmittel, Geriatrika, Hypnotika, Sedativa, Immunmodulatoren, Mundtherapeutika, Rachentherapeutika, Koronarmittel, Lipidsenker, Lokalanästhetika, Neuraltherapeutika, Magenmittel, Darmmittel, Migränemittel, Muskelrelaxantia, Narkosemittel, Neuropathiepräparate, Ophthalmika, Otologika, Parkinsonmittel, Psychopharmaka, Rhinologika, Sinusitismittel, Spasmolytika, Thrombocytenaggregationsmemmer, Tuberculosemittel, Urologica und Zystostatika ist.

6. Intravenös applizierbare, pharmazeutische Darreichungsform gemäß Anspruch 5, **dadurch gekennzeichnet, daß** der pharmazeutische Wirkstoff ausgewählt ist aus der Gruppe bestehend aus Analgetika, Analeptika, Antihypoxämika, Antiallergika, Antiarrhythmika, Antiemetika, Antivertiginosa, Antihypertonika, Antihypotonika, Antitussiva, Expectorantia, β-Rezeptorenblocker, Kalziumkanalblocker, Ophthalmika, Otologika, Spasmolytika und Urologica, vorzugsweise aus der Gruppe der Analgetika.

7. Intravenös applizierbare, pharmazeutische Darreichungsform gemäß Anspruch 5 oder 6, **dadurch gekennzeichnet, daß** der pharmazeutische Wirkstoff Paracetamol ist.

8. Intravenös applizierbare, pharmazeutische Darreichungsform gemäß einem der Ansprüche 1 bis 7, **dadurch gekennzeichnet, daß** sie physiologisch verträgliche Hilfsstoffe, vorzugsweise ausgewählt aus der Gruppe bestehend aus pH-Regulatoren, Regulatoren zur Einstellung der Osmolalität, grenzflächenaktive Verbindungen, Viskositätsregulatoren, Peptisatoren, Puffer, Konservierungsmittel oder ein Gemisch aus wenigstens zwei Vertretern aus verschiedenen, vorstehend genannten Klassen oder aus wenigstens zwei Vertretern aus einer Klasse aufweist.

9. Kit, **dadurch gekennzeichnet, daß** es die Komponenten zur Herstellung einer intravenös applizierbaren, pharmazeutischen Darreichungsform gemäß einem der Ansprüche 1 bis 8 zumindest teilweilse separiert voneinander enthält.

10. Kit gemäß Anspruch 9, **dadurch gekennzeichnet, daß** es zwei Gefäße, vorzugsweise Ampullen, Flaschen (vials) und/oder Beutel umfaßt, welche vorzugsweise aneinander andockbar sind.

11. Kit gemäß Anspruch 9, **dadurch gekennzeichnet, daß** es in Form einer Zweikammerspritze vorliegt, wobei die eine Kammer das Suspensionsmedium und die andere Kammer die zu suspendierenden Wirkstoffpartikel enthält.

## Claims

1. An intravenously administrable pharmaceutical dosage form, **characterised in that** it comprises one or more pharmaceutical active ingredients in each case at least partially undissolved in a water-based suspension medium and this undissolved fraction of active ingredient has an average particle size of > 5 µm, wherein the fraction of particles with a particle size in the range of larger than 2 µm - 100 µm is at least 80% of the total mass of these particles, and this undissolved active ingredient fraction is at most of a size such that on dilution with up to 500 ml of phosphate buffer solution at body temperature it dissolves within a predetermined administration time.

2. Intravenously administrable pharmaceutical dosage form according to claim 1, **characterised in that** it is suitable for intravenous injection.

3. Intravenously administrable pharmaceutical dosage form according to claim 1 or 2, **characterised in that** the average particle size of the undissolved active ingredient particles is in the range of > 5 to 35 µm, wherein the fraction of particles with a particle size in the range of 3-80 µm, preferably 3-50 µm, amounts to at least 80% of the total mass of these particles.

4. Intravenously administrable pharmaceutical dosage form according to one of claims 1 to 3, **characterised in that** the active ingredient particles are suspended in the suspension medium preferably immediately before administration to the patient.

5. Intravenously administrable pharmaceutical dosage form according to one of claims 1 to 4, **characterised in that** the pharmaceutical active ingredient is at least one active ingredient selected from the group comprising analgesics, antiadipose agents, analeptics, antihypoxaemic agents, antirheumatics, opioid antagonists, anthelmintics, antiallergics, antiarrhythmics, antibiotics, antidementia agents (nootropics), antidiabetic agents, antiemetics, antivertigo agents, antiepileptics, antihypertensives, antihypotensives, antimycotics, antiinflammatory agents, antitussives, expectorants, antiarteriosclerotics, β-receptor blockers, calcium channel blockers, broncholytics, antiasthma agents, cholinergics, diuretics, circulation-promoting agents, antiaddiction agents, geriatric agents, hypnotics, sedatives, immunomodulators, oral therapeutic agents, pharyngeal therapeutic agents, coronary agents, lipid-reducing agents, local anaesthetics, neural therapeutic agents, gastric agents, intestinal agents, migraine agents, muscle relaxants, narcotics, neuropathy preparations, ophthalmic agents, otological agents, antiparkinson agents, psychopharmaceuticals, rhinological agents, sinusitis agents, spasmolytics, thrombocyte aggregation inhibitors, antituberculosis agents, urological and cytostatic agents.

6. Intravenously administrable pharmaceutical dosage form according to claim 5, **characterised in that** the pharmaceutical active ingredient is selected from the group comprising analgesics, analeptics, antihypoxaemic agents, antiallergics, antiarrhythmics, antiemetics, antivertigo agents, antihypertensives, antihypotensives, antitussives, expectorants, β-receptor blockers, calcium channel blockers, ophthalmic agents, otological agents, spasmolytic and urological agents, preferably from the group of analgesics.

7. Intravenously administrable pharmaceutical dosage form according to claim 5 or 6, **characterised in that** the pharmaceutical active ingredient is paracetamol.

8. Intravenously administrable pharmaceutical dosage form according to one of claims 1 to 7, **characterised in that** it comprises physiologically tolerable auxiliary substances, preferably selected from the group comprising pH-regulators, regulators for adjusting osmolality, surface-active compounds, viscosity regulators, peptising agents, buffers, preservatives or a mixture of at least two representatives from different above-stated classes or at least two representatives from one class.

9. A kit, **characterised in that** it contains the components for the production of an intravenously administrable pharmaceutical dosage form according to one of claims 1 to 8 at least partially separate from one another.

10. Kit according to claim 9, **characterised in that** it comprises two receptacles, preferable ampoules, vials and/or bags, which can preferably be linked to one another.

11. Kit according to claim 9, **characterised in that** it is provided in the form of a dualchamber syringe, wherein one chamber contains the suspension medium and the other chamber contains the particles of active ingredient to be suspended.

## Revendications

1. Forme d'administration pharmaceutique administrable par voie intraveineuse, **caractérisée en ce qu'**elle présente une ou plusieurs substances actives pharmaceutiques, à chaque fois sous une forme au moins partiellement non dissoute dans un milieu de suspension à base d'eau et cette proportion de substance active non dissoute présente une grosseur moyenne des particules > 5 µm, la proportion de particules présentant une grosseur de particules dans la plage supérieure à 2 µm-100 µm représentant au moins 80% de la masse totale de ces particules et cette proportion de substance active non dissoute étant au maximum telle qu'elle se dissout en un temps d'administration prédéfini lors de la dilution par jusqu'à 500 ml de solution tampon au phosphate à la température corporelle.

2. Forme d'administration pharmaceutique administrable par voie intraveineuse selon la revendication 1, **caractérisée en ce qu'**elle convient pour l'injection par voie intraveineuse.

3. Forme d'administration pharmaceutique administrable par voie intraveineuse selon la revendication 1 ou 2, **caractérisée en ce que** les grosseurs moyennes des particules de substance active non dissoutes se situe dans la plage de > 5 à 35 µm, la proportion de particules présentant une grosseur de particules dans la plage de 3-80 µm, de préférence de 3-50 µm, représentant au moins 80% de la masse totale de ces particules.

4. Forme d'administration pharmaceutique administrable par voie intraveineuse selon l'une quelconque des revendications 1 à 3, **caractérisée en ce que** les particules de substance active sont de préférence mises en suspension juste avant l'administration aux patients dans le milieu de suspension.

5. Forme d'administration pharmaceutique administrable par voie intraveineuse selon l'une quelconque des revendications 1 à 4, **caractérisée en ce que** la substance active pharmaceutique est au moins une substance active choisie dans le groupe constitué par les analgésiques, les agents contre l'obésité, les analeptiques, les agents anti-hypoxémiques, les agents antirhumatismaux, les antagonistes des opioïdes, les anthelminthiques, les antiallergiques, les antiarrhythmiques, les antibiotiques, les nootropiques, les antidiabétiques, les antiémétiques, les antivertigineux, les antiépileptiques, les anti-hypertoniques, les anti-hypotoniques, les antimycotiques, les antiphlogistiques, les antitussifs, les expectorants, les agents anti-artérioscléreux, les bloquants des récepteurs ß, les agents de blocage du canal calcique, les agents broncholytiques, les antiasthmatiques, les cholinergiques, les diurétiques, les agents favorisant la circulation sanguine, les agents de sevrage, les médicaments gériatriques, les hypnotiques, les sédatifs, les immunomodulateurs, les préparations orales, les préparations pour le pharynx, les agents thérapeutiques pour le pharynx, les agents coronaires, les antilipidémiques, les anesthésiques locaux, les agents thérapeutiques neuraux, les agents pour l'estomac, les agents pour les intestins, les antimigraineux, les relaxants musculaires, les narcotiques, les préparations pour la neuropathie, les agents ophtalmiques, les agents otologiques, les agents antiparkinsoniens, les produits psychopharmaceutiques, les agents contre les rhinites, contre les sinusites, les spasmolytiques, les inhibiteurs de l'agrégation des thrombocytes, les agents contre la tuberculose, les agents urologiques et les cytostatiques.

6. Forme d'administration pharmaceutique administrable par voie intraveineuse selon la revendication 5, **caractérisée en ce que** la substance active pharmaceutique est choisie dans le groupe constitué par les analgésiques, les analeptiques, les anti-hypoxémiques, les antiallergiques, les agents antiarrhythmiques, les antiémétiques, les antivertigineux, les anti-hypertoniques, les anti-hypotoniques, les antitussifs, les expectorants, les bloquants des récepteurs ß, les agents de blocage du canal calcique, les agents ophtalmiques, les agents otologiques, les spasmolytiques et les agents urologiques, de préférence ceux du groupe de analgésiques.

7. Forme d'administration pharmaceutique administrable par voie intraveineuse selon la revendication 5 ou 6, **caractérisée en ce que** la substance active pharmaceutique est le paracétamol.

8. Forme d'administration pharmaceutique administrable par voie intraveineuse selon l'une quelconque des revendications 1 à 7, **caractérisée en ce qu'**elle présente des adjuvants physiologiquement acceptables, de préférence choisis dans le groupe constitué par les régulateurs du pH, les régulateurs pour le réglage de l'osmolalité, les composés tensioactifs, les régulateurs de la viscosité, les agents peptisants, les tampons, les conservateurs ou un mélange d'au moins deux représentants des différentes classes susmentionnées ou d'au moins deux représentants d'une classe.

9. Kit, **caractérisé en ce qu'**il contient les composants pour la préparation d'une forme d'administration pharmaceutique administrable par voie intraveineuse selon l'une quelconque des revendications 1 à 8 sous une forme au moins partiellement séparée les uns des autres.

10. Kit selon la revendication 9, **caractérisé en ce qu'**il comprend deux récipients, de préférence des ampoules, des flacons (vials) et/ou des sachets, qui sont de préférence fixables les uns aux autres.

11. Kit selon la revendication 9, **caractérisé en ce qu'**il se trouve sous forme d'une seringue à deux chambres, dont une chambre contient le milieu de suspension et l'autre chambre les particules de substance active à mettre en suspension.
